Europäisches Patentamt

(19) European Patent Office

Office européen des brevets

(11) Publication number : **0 367 586 B1**

(12) **EUROPEAN PATENT SPECIFICATION**

(45) Date of publication of patent specification :
17.06.92 Bulletin 92/25

(51) Int. Cl.⁵ : **A61K 31/71, A61K 9/08,
A61K 47/18, A61K 7/48**

(21) Application number : **89311289.6**

(22) Date of filing : **01.11.89**

(54) **Primycin solutions.**

(30) Priority : **02.11.88 HU 566988**

(43) Date of publication of application :
**09.05.90 Bulletin 90/19**

(45) Publication of the grant of the patent :
**17.06.92 Bulletin 92/25**

(84) Designated Contracting States :
**AT BE CH DE FR GB IT LI NL SE**

(56) References cited :
**EP-A- 0 224 868
FR-A- 2 315 910**

(73) Proprietor : **CHINOIN Gyogyszer és
Vegyészeti Termékek Gyára RT.
To utca 1-5
H-1045 Budapest IV (HU)**

(72) Inventor : **Szentmiklosi, Peter
Rudas L u 75
H-1064 Budapest (HU)**
Inventor : **Szuts, Tamas
Csalogany u 53
H-1027 Budapest (HU)**
Inventor : **Hidasi, Gyorgy
Sarret Park 7/a. I/4
H-1142 Budapest (HU)**
Inventor : **Juhasz, Istvan
Bartok B 3/F
H-1225 Budapest (HU)**

(74) Representative : **Skailes, Humphrey John et al
Frank B. Dehn & Co. Imperial House 15-19
Kingsway
London WC2B 6UZ (GB)**

## Description

The invention relates to stable aqueous solutions of primycin which are useful for a topical therapeutic treatment in itself or in liquid or semisolid compositions. The invention further relates to the preparation of the aqueous solution and the compositions.

The antibiotic known as "primycin" has first been described in the Hungarian patent specification No. 153,539; it was extracted as a native antibiotic from the product of a fermentation carried out with the culture of Thermopolyspora galeriensis fungal strain. Primycin mainly acts on gram-positive cocci, is not absorbable and is useful for a topical treatment. The preparation of primycin has been published in J. Chem Soc. Perkin I 1984, 816 wherein primycin was characterized by a single formula. However, it has since then become known that this substance is a mixture consisting of several components (cf. the Hungarian patent specifications Nos. 195,514 and 196,425). Although primycin is a very effective antibiotic, its therapeutical utilization was made extraordinarily difficult since it is practically insoluble either in water or in physiologically acceptable solvents. Thus, several efforts had earlier been made to eliminate these difficulties and to prepare pharmaceutical compositions containing primycin in a therapeutically well utilizable form while maintaining its efficiency as a whole.

Thus, the preparation of a semisolid composition containing primycin as a heterocolloid in 50% aqueous ethanol was suggested in the Hungarian patent specification No. 173,708. However, by using this process, a gel containing at most 0.2% of primycin can be prepared with the further disadvantage that a burning pain sensation on the damaged skin surface is elicited. This has to be avoided by adding a local anesthetic agent to the composition whereby an allergic response can be evoked in the patient. A further disadvantage consists in that skin damage can occur by a long-lasting treatment with such a composition as has been proved by animal experiments carried out on New Zealand rabbits.

A composition containing the complex of primycin sulfate with N-methyl-pyrrolidone in a predetermined weight ratio and suggested in the Hungarian patent specification No. 194,493 proved to be preferred. This composition having a gel consistency is useful to incorporate the active ingredient as a micro-distribution in practically all galenic forms such as ointments, creams, foams and dusting powders, however, it is not useful to prepare transparent solutions.

There exists, however, an area of the dermatology which requires an antibiotic treatment, i.e. the skin disease caused by Propionibacterium acne, the various types of which are usually treated by painting with alcoholic solutions of antibiotics namely, both the patient and the disease require the cleaning and drying effects of alcohol. Since this treatment does not involve any prolonged exposure (as with the semisolid heterocolloid containing alcohol), this painting with an alcoholic can be used without any danger.

For application as alcoholic solutions, antibiotics with a tetracycline--skeleton as well as erythromycins have been used for a long time; however, most of the pathogens became resistant to these antibiotics. Due to its very high price, clindamycin introduced in the recent years could not be widely used.

The aqueous alcohol-based compositions of this kind known up to the present usually contain about 50% of alcohol and 1 to 1.5 % by mass of antibiotic dissolved therein such as Staticin 1.5 topical solution (Physician Desk Reference 40, Ed. Medical E. Comp. USA 1986).

Primycin destroying in a concentration of as low as $10^{-7}$ g/ml both the Propionibacterium acne as well as staphylococcus bacteria, being present as co-pathogens, would be considered as an ideal antibiotic for the topical therapy of acne if a solution of at least 1% concentration could be prepared. However, such solution could not till now be prepared from primycin since primycin sulfate dissolves a maximum of 0.2% in ethanol or propanol. This problem is solved by the process according to the present invention which results without any difficulty in a solution containing primycin in a concentration of even 1.5% by weight.

In the course of these investigations, it has suprisingly been recognized that, on mixing a salt or an other suitable derivative of primycin with an appropriate amount of pyroglutamic acid in isopropanol under heating, a milky suspension in obtained which is transformed to a limpid, stable solution after dilution with water while continuously stirring under heating. The solution thus obtained remains limpid and stable after cooling down and can be used for therapeutic treatments, mainly for topical treatments; but it is useful also for the preparation of topically used common pharmaceutical compositions such as gels, foams and aerosols by using known methods of formulation and pharmaceutically acceptable additives.

This invention is novel and highly surprising since the process can be accomplished by using only the adequate weight ratios of the substance mentioned and specifically by using the substances mentioned above. Thus, isopropanol can not be replaced by other alcohols or solvents since no suspension, convertible to an aqueous solution can be obtained by using e.g. methanol, ethanol, butanol, octanol or polyvalent alcohols such as ethylene glycol or glycerol; and similarly, pyroglutamic acid cannot be replaced by other amino acids, e.g. glutamine, asparagine, alanine or glycine. Similarly, no satisfactory results are obtained when other amounts,

2

e.g. an other concentration of isopropanol are used which are different from the preferred weight ratios to be described hereinafter.

Namely, it has been found that a clear, stable solution can be obtained only in the case when 0.5 to 1.75% by weight/volume of primycin sulfate and 2 to 15 % by mass/volume of pyroglutamic acid (optionally in the form of one of its soluble salts, preferably the sodium salt) as calculated for the volume of the solution to be prepared, are suspended while stirring and heating suitably at a temperature of 40 to 60 °C to obtain a solution containing 40 to 60 % by volume of isopropanol after dilution with water.

Thus, the present invention relates to novel, stable aqueous primycin solutions containing 0.5 to 1.75% by weight/volume of primycin sulfate and 2 to 15 % by weight/volume of pyroglutamic acid, preferably L-pyroglutamic acid or a soluble salt, preferably the sodium salt thereof as calculated for the volume of the solution to be prepared and 40 to 60% by volume of isopropanol as calculated for the volume of the solution to be prepared as well as water in an amount supplementing up to 100%.

The invention further relates to a process for the preparation of the above novel stable aqueous solutions, which comprises suspending 0.5 to 1.75% by weight/volume of primycin sulfate and 2 to 15% by weight/volume of pyroglutamic acid, preferably L-pyroglutamic acid or a soluble salt, preferably the sodium salt thereof as calculated for the volume of the solution to be prepared while stirring and heating suitably at a temperature of 40 to 60 °C, diluting the milky suspension thus obtained for the desired concentration with water while stirring, then cooling down and, if desired, transforming the solution thus obtained to a topically applicable pharmaceutical or disinfecting cosmetic composition by adding the usual pharmaceutical or cosmetical auxiliary materials and optionally other therapeutically active, suitably desquamating and/or antiinflammatory agent(s).

According to a preferred embodiment of the invention, primycin is used as a complex formed with N-methylpyrrolidone mentioned above instead of its sulfate to obtain stable solutions with very advantageous properties. Preferably, a complex containing 20% by weight of primycin is used in an amount of 2.5 to 9% by weight/volume for the preparation of the stable aqueous solution according to the invention.

The clear, stable solutions according to the invention can be formulated to topically applicable pharmaceutical compositions by using known techniques. Disinfecting cosmetic compositions such as ointments, creams, foams and aerosols or animal food concentrates (for veterinary medicinal use) can similarly be prepared by using the above solutions. In the course of preparing these compositions, a therapeutically useful preparation containing the primycin salt or the complex formed from primycin with N-methyl-pyrrolidone can also be used: e.g. the Ebrimycin NMP gel (Chinoin, Budapest) based on the Hungarian patent specification No. 194,493 mentioned hereinabove is useful for this purpose.

In the composition according to the invention, pyroglutamic acid can also be used in the form of a preparation optionally containing therapeutically and/or cosmetically preferable additives. Pyroglutamic acid may e.g. conveniently be used in the form of Lactil[R], a composition of Th. Goldschmidt AG. (German Federal Republic) containing cosmetically or dermatologically preferred additives such as collagen and carbohydrates in addition to 25% by weight of sodium pyroglutamate (see, "Lexikon der Hilfstoffe" Vol. II, Ed. Cantor. Aulendorf, page 511, 1981).

The invention is illustrated in detail by the following Examples.

## Example 1

1.0 g of primycin sulfate and 8.0 g of 1-pyroglutamic acid are suspended in 50 ml of isopropanol at 50 °C in a water bath while vigorous stirring, then the milky suspension is filled up to 100 ml with distilled water at the same temperature under vigorous stirring and allowed to cool down to room temperature to obtain a limpid, stable solution.

## Example 2

5.0 g of a complex of primycin with N-methylpyrrolidone (containing 1.0 g of primycin) and 8.0 g of L-pyroglutamic acid are transformed to a suspension with 50 ml of isopropanol at 50 °C in a water bath while vigorously stirring, then the milky suspension thus obtained is filled up to 100 ml with distilled water under vigorous stirring and cooled down to room temperature to obtain a limpid, stable solution.

## Example 3

The process described in Example 1 is followed, except that 10.0 ml of Lactyl[R] (containing 2.5 g of pyroglutamic acid) are used instead of 8.0 g of L-pyroglutamic acid. A limpid, stable solution is obtained.

Example 4

The process described in Example 1 is followed by using 7.5 g of the complex of primycin with N-methyl-pyrrolidone (containing 1.5 g of primycin) and 10.0 ml of Lactyl[R] (containing 2.5 g of pyroglutamic acid) as starting substances to give a limpid, stable solution.

Example 5

The process described in Example 1 is followed by using 1.5 g of primycin sulfate and 9.0 g of sodium L-pyroglutamate as starting substances to obtain a limpid, stable solution.

Example 6

After suspending 1 g of primycin sulfate and 14 g of DL-pyroglutamic acid in 45 ml of isopropanol at 50 °C in a water bath under vigorous stirring, the milky suspension thus obtained is filled up to 100 ml with distilled water at the same temperature while stirring vigorously to give a limpid, stable solution.

Example 7

Preparation of an aerosol

After melting 5.0 of Ebrimycin NMP[R] gel (containing 1.0 g of primycin) together with 8.0 g of L-pyroglutamic acid at 60 °C while stirring, 52.0 g of isopropanol are gradually added, then the mixture is filled up to 100 ml with distilled water and maintained at 60 °C under constant stirring until a clear solution is obtained.

10 parts by mass of Frigen® 11/12 expelling gas are compressed with 90 parts by weight of the above solution cooled to room temperature in an aerosol bottle by using a suitable filling system.

Instead of the Frigen® expelling gas, the aerosol can be prepared also with a propane-butane expelling gas by using appropriate equipment.

Example 8

Preparation of a foam

For the preparation of an emulsion which may be used as a foam, the solutions are prepared in two phases (phases "A" and "B", respectively), which are composed as follows.

Phase "A": Ebrimycin NMP[R] gel (containing 20% by weight of prymicin)     3,75

Lactil[R] (containing 25% by weight of pyroglutamic acid)    7,50g

Isopropanol    7,50g

Distilled water    26.00g

Phase "B": Paraffin oil    5.00g

Isopropyl myristate    5.00g

Emulgator ®  E 2149 (Goldschmidt, German Federal Republic)    5.00g

Stearin    10.25g

Both phase are separatively homogenized at 60 °C, then phase "A" is poured as a thin stream in to phase "B" and the mixture obtained is stirred until it is cooled down. The emulsion thus prepared is filled into a foam-spraying bottle together with the expelling gas.

Example 9

Preparation of a gel
A gel is prepared from the solution according to the invention by using the following components.

| | |
|---|---|
| Ebrimycin NMP[R] gel | 4.50 g |
| Lactil® | 9.00 g |
| Isopropanol | 45.00 g |
| Distilled water | 31.50 g |
| Carbopol® 940 | 2.00 g |
| Triethanolamine | 2.00 g |
| Tagat® L (Goldschmidt) | 4.00 g |
| Tagat® M (Goldschmidt) | 2.00 g |

As described above, Ebrimycin NMP[R] gel is stirred with Lactil[R] and the solvents at 60 °C, then Carbopol 940 composition is mixed to. After clearing up of the solution, the mixture of Tagat® L, Tagat® M and triethanolamine previously melted together powed into to the solution in a thin stream under constant stirring, then the mixture obtained is further stirred until it achieves a gel consistency.

Example 10

Preparation of an animal food concentrate for therapeutic use

The solution prepared according to Example 1 is applied onto a carrier which is useful for consumption by animals such as corn-grits, wheat-grits, bentonite, aerosil, talc or calcium carbonate in a fluidizing apparatus or in any other equipment which may be used for atomization, then dried at a temperature of 20 to 40 °C preferably under reduced pressure. In the course of this process, the active ingredient content of the animal food concentrate is adjusted to between 0.1 and 0.5%.

**Claims**

1. Stable aqueous primycin solutions, which comprise 0.5 to 1.75% by weight/volume of primycin sulfate or 2.5 to 9% by weight/volume of a complex of primycin with N-methylpyrrolidone and 2 to 15% by weight/volume of pyroglutamic acid, preferably L-pyroglutamic acid, or a soluble salt, preferably the sodium salt thereof, as calculated for the volume of the solution to be prepared and 40 to 60% by volume of isopropanol as calculated for the volume of the solution to be prepared, as well as water in an amount supplementing up to 100%.

2. A primycin solution as claimed in claim 1, which comprises L-pyroglutamic acid in the form of an L-pyroglutamic acid composition containing pharmaceutically acceptable additives.

3. Pharmaceutical or disinfecting cosmetic compositions mainly for topical treatment, which comprise a primycin solution as claimed in claim 1 or claim 2 optionally in admixture with pharmaceutically acceptable auxiliary materials and, if desired, with other non-synergistic therapeutically active ingredient(s), suitably desquamating and/or antiinflammatory agent(s).

4. Process for the preparation of stable aqueous primycin solutions, which comprises suspending 0.5 to 1.75% by weight/volume of primycin sulfate or 2.5 to 9% by weight/volume of a complex of primycin with N-methyl-pyrrolidone and 2 to 15% by weight/volume of pyroglutamic acid, preferably L-pyroglutamic acid, or a soluble salt, preferably the sodium salt thereof, as calculated for the volume of the solution to be prepared in 40 to 60% by volume of isopropanol as calculated volume of the solution to be prepared, while stirring and heating, and then, after dilution with water to the desired concentration, stirring the milky suspension thus obtained and cooling it down.

5. A process as claimed in claim 4, which comprises preparing the suspension at a temperature of 40 to 60 °C while stirring.

**Patentansprüche**

1. Stabile wäßrige Primycin-Lösungen, dadurch gekennzeichnet, daß sie

```
0,5 -  1,75 Gew.%/Vol. Primycin-sulfat oder
2,5 -  9    Gew.%/Vol. eines Komplexes von Primycin mit
                       N-Methylpyrrolidon und
2   - 15    Gew.%/Vol. Pyroglutaminsäure,
```

vorzugsweise L-Pyroglutaminsäure oder eines ihrer löslichen Salze, vorzugsweise des Natrium-salzes - auf das Volumen der herzustellenden Lösung berechnet - und 40 - 60 Vol.% Isopropanol - auf das Volumen der herzustellenden Lösung berechnet - sowie Wasser in der zur Ergänzung auf 100 % erforderlichen Menge enthalten.

2. Primycin-Lösung gemäß Anspruch 1, dadurch gekennzeichnet, daß sie die L-Pyroglutaminsäure in Form einer L-Pyroglutaminsäure-Komposition mit pharmazeutisch verträglichen Additiven enthält.

3. Pharmazeutische oder desinfizierend wirkende kosmetische Kompositionen, hauptsächlich für die topische Behandlung, dadurch gekennzeichnet, daß sie eine Primycinlösung gemäß Anspruch 1 oder 2, gegebenenfalls in Mischung mit pharmazeutisch verträglichen Hilfsmitteln und - falls gewünscht - mit einem oder mehreren weiteren nicht-synergistischen, therapeutisch aktiven Ingredienten, zweckmäßig Mitteln zur Desquamation und/oder entzündungshemmende Mittel enthalten.

4. Verfahren zur Herstellung stabiler wäßriger Primycin-Lösungen, dadurch gekennzeichnet, daß man

```
0,5 -  1,75 Gew.%/Vol. Primycin-sulfat oder
2,5 -  9    Gew.%/Vol. eines Komplexes von Primycin mit
                       N-Methylpyrrolidon und
2   - 15    Gew.%/Vol. Pyroglutaminsäure,
```

vorzugsweise L-Pyroglutaminsäure oder eines ihrer löslichen Salze, vorzugsweise das Natriumsalz - auf das Volumen der herzustellenden Lösung berechnet - in 40 - 60 Vol.% Isopropanol -auf das Volumen der herzustellenden Lösung berechnet - unter Rühren und Erhitzen suspendiert, und dann die nach dem Verdünnen mit Wasser auf die gwünschte Konzentration erhaltene milchige Suspension rührt und abkühlt.

5. Verfahren nach Anspruch 4, dadurch gekennzeichnet, daß man die Herstellung der Suspension bei einer Temperatur von 40 - 60°C unter Rühren durchführt.

**Revendications**

1. Solutions aqueuses stables de primycine, comprenant 0,5 à 1,75% en poids/volume de sulfate de primycine ou 2,5 à 9% en poids/volume d'un complexe de primycine avec de la N-méthylpyrrolidone et 2 à 15% en poids/volume d'acide pyroglutamique, de préférence de l'acide L-pyroglutamique, ou un sel soluble, de préférence le sel de sodium de celui-ci, sur la base du volume de la solution à préparer, et 40 à 60% en volume d'isopropanol sur la base du volume de la solution à préparer, ainsi que de l'eau en une quantité assurant le complément à 100%.

2. Solution de primycine selon la revendication 1, comprenant de l'acide L-pyroglutamique sous la forme d'une composition d'acide L-pyroglutamique contenant des additifs pharmaceutiquement acceptables.

3. Compositions pharmaceutiques ou cosmétiques désinfectantes, principalement pour traitement topique, comprenant une solution de primycine selon l'une des revendications 1 ou 2, en option en mélange avec des matières auxiliaires pharmaceutiquement acceptables et, si on le souhaite, avec au moins un autre constituant thérapeutiquement actif non-synergique, agent de désquamation approprié et/ou agent antiinflammatoire.

4. Procédé pour la préparation de solutions aqueuses stables de primycine, comprenant la mise en suspension de 0,5 à 1,75% en poids/volume de sulfate de primycine ou 2,5 à 9% en poids/volume d'un complexe de primycine avec de la N-méthyl-pyrrolidone et 2 à 15% en poids/ volume d'acide pyroglutamique, de préférence de l'acide L-pyroglutamique, ou un sel soluble, de préférence le sel de sodium de celui-ci, sur la base du volume de la solution à préparer dans 40 à 60% en volume d'isopropanol sur la base du volume de la solution à préparer, tout en agitant et chauffant, et ensuite, après dilution avec de l'eau jusqu'à la concentration désirée, l'agitation de la suspension laiteuse ainsi obtenue et son refroidissement.

5. Procédé selon la revendication 4, comprenant la préparation de la suspension à une température de 40 à 60°C sous agitation.